# EUROPEAN PATENT APPLICATION

(11) **EP 4 321 614 A1**
(43) Date of publication of application: **14.02.2024**
(21) Application number: 22929496.2
(22) Date of filing: 05.07.2022
(51) Int. Cl.: C12N 5/079, A61K 35/30, A61P 25/00, A61P 9/10, A61P 25/28, A61P 25/16

(54) **METHOD FOR PREPARING OLIGODENDROCYTES AND USE**

(30) Priority: 02.03.2022 CN 202210194839
(71) Applicant: Shenzhen Exoneuglia Biomedical Technology Co., Ltd., Shenzhen, Guangdong 518107 (CN)
(72) Inventor: GUO, Ying, Shenzhen, Guangdong 518107 (CN); GAO, Hong, Shenzhen, Guangdong 518107 (CN)
(74) Representative: ZBM Patents - Zea, Barlocci & Markvardsen
(86) International application number: PCT/CN2022/103803
(87) International publication number: WO 2023/165062

(57) **Abstract**

The present invention provides a method of producing oligodendrocytes and the application thereof. The protocol provided recapitulates the major steps of oligodendrocyte differentiation in a significantly shorter time than the 75-95 days required for the previous protocol. The cannabinoid receptor agonist, WIN55212-2, induces NSC differentiation and promotes OLIG2+OPC induction. WIN-derived OLIG2+ progenitor cells differentiate into PDGFR α+ OPCs, which are highly migratory, can further differentiate into mature OLs with strong myelination ability. When WIN-derived OPCs were transplanted to the lesion site, the motor ability of spinal cord injury (SCI) mice was significantly improved from the second week after transplantation. Immunostaining results showed that WIN-derived OPCs could differentiate into mature OLs and myelinate injured axons. The method and product of the present invention have wide applications in the field of cellular therapy, especially for spinal cord injury with remarkable therapeutic effects.

## Description

### Technical field

The invention belongs to the field of stem cell therapy, and particularly relates to a method of producing oligodendrocytes and the application thereof.

### Background

Human induced pluripotent stem cells (iPSCs) can be generated by reprogramming somatic cells such as human skin, blood and others *in vitro,* and have an infinite proliferation ability similar to human embryonic stem cells (ESCs), as well as an ability of differentiating into almost all functional cells *in vitro,* including neural stem cells (NSCs). This characteristic of iPSC successfully bypasses ethical issues and has a wide range of applications in terms of stem cell therapy.

Oligodendrocytes (OLs) are cells of the central nervous system that exist in vertebrates. They produce lipid-rich lamellar myelin sheaths that cover neuronal axons and generate defined electrically insulated segments to maximize action potential conduction velocity. Myelin is also important for axonal integrity and survival, and it has been shown that even small changes affecting oligodendrocyte metabolism can lead to neurodegeneration.

Animal experiments have demonstrated that transplanted oligodendrocyte precursor cells (OPCs) can promote white matter retention, increase the number of endogenous oligodendrocytes, reduce cavity volume, and thereby improve motor recovery. The potential therapeutic mechanism of OPC may be remyelination, modulation of the local immune microenvironment, secretion of neurotrophic factors, and provision of a physical scaffold to support the growing axons. OPC can secrete a series of substances, including growth factors, neurotrophic factors, chemokines, and cytokines.

Spinal cord injury (SCI) is a devastating disease that leads to local nerve deficiency or demyelination. However, the remyelination process mediated by endogenous oligodendrocyte precursor cells (OPCs) is insufficient. Therefore, transplantation of exogenous OPC is considered to be an effective method for achieving remyelination in SCI models. However, most of the current methods for generating OPC are time-consuming or based on lentiviral techniques, which suffer from low efficiency or safety problems.

WIN55212-2, a CB receptor agonist, was found to be a potent analgesic in rat neuropathic pain models. It activates p42 and p44 MAP kinases via receptor-mediated signaling.

### Summary of invention

The first aspect of the present invention provides a method for preparing OLIG2+ neural stem cells (NSCs), which is characterized by comprising the following steps:
(1) Generating NSCs of PAX6+/NESTIN+/SOX2+: PSCs were cultured with neural induction medium from day 0 to day 7, and the medium was changed every day; Preferably, the PSC confluence rate is 70-90%;
(2) From day 8, changing the medium to fresh N2 medium containing retinoic acid (RA), Purmorphamine (Pur) and CB receptor agonist every day for 4-5 days;
Preferably, OLIG2+ neural stem cells reached at least 90% at day 12; more preferably, OLIG2+ neural stem cells reached at least 95% at day 12.

The second aspect of the present invention provides a method for preparing oligodendrocyte precursor cells (OPCs), which is characterized by comprising the following steps:
(1) Generating NSCs of PAX6+/NESTIN+/SOX2+: PSCs were cultured with neural induction medium from day 0 to day 7, and the medium was changed every day; preferably, the PSC cell density is 70-90%;
(2) From day 8, changing the medium to fresh N2 medium containing RA, Pur and CB receptor agonist every day;
(3) From day 12, manually isolating the cells and suspending them in N2B27 medium containing RA, PUR and CB receptor agonist, and changing the medium every other day;
(4) From day 20, further suspending spheroids in PDGF-AA medium;
(5) From day 30, seeding and culturing expanded spheroids on Matrigel-coated plates or Petri dishes with low growth factors in glial differentiation medium for 3-15 days;

Preferably, the OPC of PDGFR α+ reached at least 90% at day 34; more preferably, the OPC of PDGFR α+ reached at least 95% at day 34;
Preferably, the OPC of O4+ reached at least 35% at day 44; more preferably, the OPC of O4+ reached at least 40% at day 44.

The third aspect of the present invention provides a method for preparing oligodendrocytes (OLs), which is characterized by comprising the following steps:
(1) Generating NSCs of PAX6+/NESTIN+/SOX2+: PSCs were cultured with neural induction medium from day 0 to day 7, and the medium was changed every day; Preferably, the PSC cell density is 70-90%;
(2) From day 8, changing the medium to fresh N2 medium containing RA, Pur and CB receptor agonist every day;
(3) From day 12, manually isolating the cells and suspending them in N2B27 medium containing RA, PUR and CB receptor agonist, and changing the medium every other day;
(4) From day 20, further suspending spheroids in PDGF-AA medium;
(5) From day 30, seeding and culturing expanded spheroids on Matrigel-coated plates or Petri dishes with low growth factors in glial differentiation medium for 16-36 days;
Preferably, the OLs coexpressing O4 and MBP reached at least 30% at day 66; more preferably, the OLs coexpressing O4 and MBP reached at least 35% at day 66.

According to the methods described in the above three aspects, characterized in that step (1) is replaced with the NSC finished product of PAX6+/NESTIN+/SOX2+.

According to the methods described in the above three aspects, characterized in that the PSCs are cultured on Matrigel-coated six-well plates using mTeSR^{™} medium (stem cell technologies, Canada) in a 37 °C incubator with 5% CO₂.

Preferably, The PSC is hPSC. The PSC includes ESC and iPSC, preferably hESC and hiPSC. The PSC can be seeded on a surface containing a matrix, such as a gel or a basement membrane matrix.

According to the methods described in the above three aspects, characterized in that the concentration of the RA is 10-1000nM, the concentration of the Pur is 0.1-10 µM, and the concentration of the CB receptor agonist is 0.1-10 µM.

According to the methods described in the above three aspects, characterized in that the concentration of the RA is 100 nM, the concentration of the Pur is 1 µM, and the concentration of the CB receptor agonist is 1 µM.

According to the methods described in the above three aspects, characterized in that the CB receptor agonist is selected from one or more of CP55940, AM-1241, JVH-015 (and its similar structures JVH-151, JVH-120), A-796260, L-768242, resunab, ACPA (Arachidonylcyclopropylamide), 2-Arachidonoyl glycerol-d8, Bay 59-3074, BML-190 (IMMA), GW842166X, Noladin ether and Gp 1a.

According to the methods described in the above three aspects, characterized in that the CB receptor agonist is WIN55212-2.

The fourth aspect of the invention discloses an oligodendrocyte precursor cell, which is characterized in that it is prepared according to the method described in the second aspect.

The fifth aspect of the invention discloses an oligodendrocyte, which is characterized in that it is prepared according to the method described in the third aspect.

The sixth aspect of the invention discloses use of said oligodendrocyte precursor cell or said oligodendrocyte in the preparation of medicament for the treatment of demyelinating and myelin sheath injury diseases.

Preferably, the demyelinating diseases include spinal cord injury, stroke, multiple sclerosis, neuromyelitis optica, Guillain-Barre syndrome, diffuse disseminated cerebrospinal meningitis, acute disseminated encephalomyelitis, concentric sclerosis, diffuse sclerosis, leukodystrophy, leukoencephalopathy caused by ischemia-hypoxia disease, central pontine myelinolysis, acute inflammatory demyelinating polyneuropathy, chronic inflammatory demyelinating polyneuropathy, subacute combined degeneration caused by nutritional deficiency disease, subacute sclerosing panencephalitis or progressive multifocal leukoencephalopathy caused by viral infection, diabetic neuropathy, neuropathy of systemic lupus erythematosus, white matter ablation disease, adrenoleukodystrophy, and pelizois metzbach disease.

Preferably, the myelin sheath injury diseases include Down syndrome, Alzheimer's disease, Parkinson's disease, and other neurological diseases.

Down syndrome, Alzheimer's disease, Parkinson's disease and other neurological diseases can cause obvious myelin sheath injury.

The seventh aspect of the invention discloses a medium for promoting NSC differentiation, the components of which comprise fresh N2 medium containing retinoic acid (RA), Purmorphamine (Pur) and CB receptor agonist.

Preferably, the concentration of the RA is 10-1000nM, the concentration of the Pur is 0.1-10 µM, and the concentration of the CB receptor agonist is 0.1-10 µM; more preferably, the concentration of the RA is 100 nM, the concentration of the Pur is 1 µM, and the concentration of the CB receptor agonist is 1 µM.

The eighth aspect of the invention discloses a medium for promoting NSC differentiation, the components of which comprise fresh N2B27 medium containing retinoic acid (RA), Purmorphamine (Pur) and CB receptor agonist.

Preferably, the concentration of the RA is 10-1000nM, the concentration of the Pur is 0.1-10 µM, and the concentration of the CB receptor agonist is 0.1-10 µM; more preferably, the concentration of the RA is 100 nM, the concentration of the Pur is 1 µM, and the concentration of the CB receptor agonist is 1 µM.

The ninth aspect of the present invention discloses use of the above medium for promoting NSC differentiation.

Compared with the prior technology, the invention has achieved remarkable technical effects:

### (1) WIN55212-2 promoted hiPSC-derived NSCs to express OLIG2 in a CB1/CB2 dependent manner

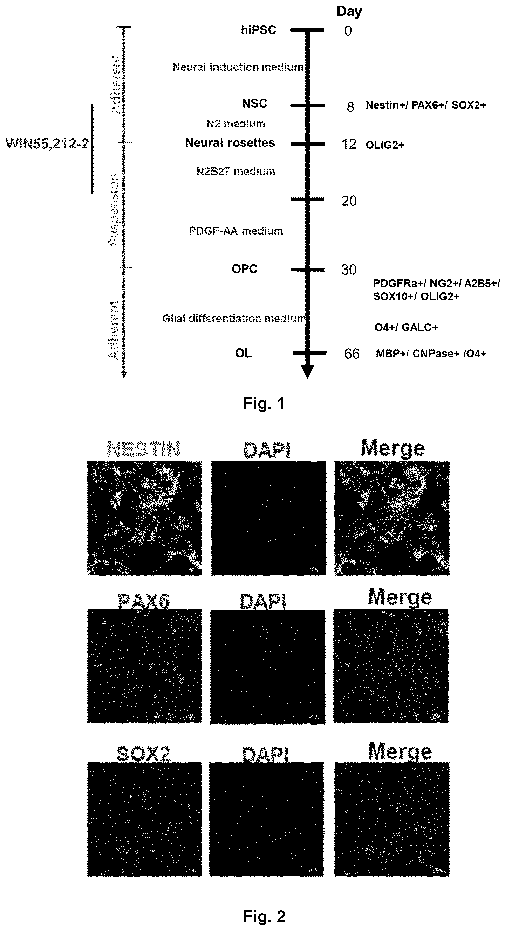

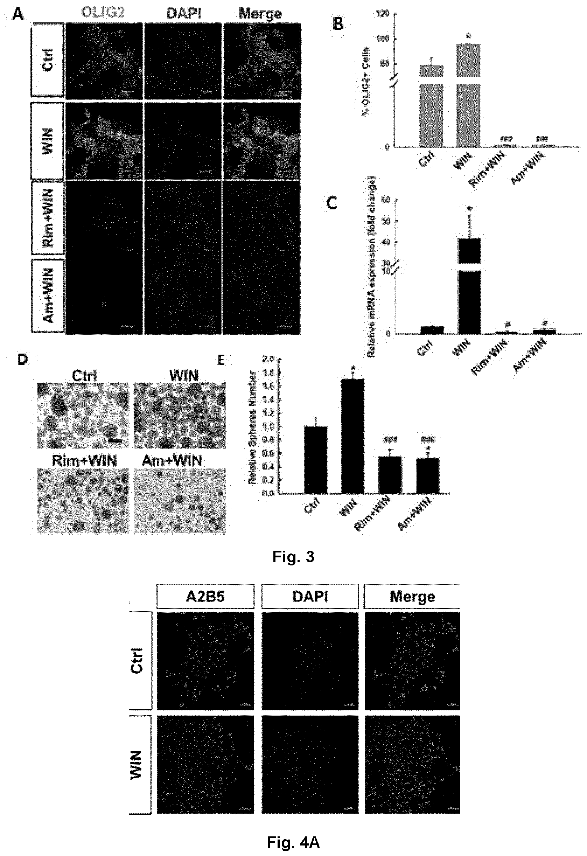

NSCs expressing PAX6, NESTIN, and SOX2 were generated from hiPSC using the dual SMAD neural induction method (Fig. 2). When treated with a series of concentrations of WINs, the inventors found that the concentration of 1 µM is a safe concentration and has no side effects on the growth of NSC, 1 µM WIN greatly increased the production of OLIG2-positive (OLIG2+) cells in NSCs, and 95.4 ± 0.2% of cells OLIG2 + was more than those in the control group (78.8 ± 5.8%, *n* =3, P <0.05) at D12 (A and B in Fig. 3). The upregulation effect was significantly inhibited by CB1 inhibitor, rimonabant, or CB2 inhibitor, AM630 (A, B, and C in Fig. 3). Similar results were also observed in OLIG2 mRNA validated by QRT - PCR (C of Fig. 3).

When cells were detected in suspension cultures, OLIG2-negative cells were eliminated by medium changing because these cells did not form aggregates (Douvaras, P. and V. Fossati, Generation and isolation of oligodendrocyte progenitor cells from human pluripotent stem cells. Nature Protocols, 2015. 10(8): p. 1143-1154. Namchaiw, P., et al., Temporal and partial inhibition of GLI1 in neural stem cells (NSCs) results in the early maturation of NSC derived oligodendrocytes in vitro. Stem Cell Res Ther, 2019. 10(1): p. 272.). OLIG2-positive cells were enriched during this process, and the number of spheroids was determined at D20. As shown in D and E of Fig. 3, OLIG2+ spheroids in WIN treatment group were 1.7 ± 0.1 times higher than those in the control group (*n* =3, P <0.05). When co-treated with CB1 or CB2 inhibitors, OLIG2-positive spheroids were significantly reduced (*n* =3, *P* <0.001, compared with WIN Group). The results showed that WIN promoted not only the expression of OLIG2, but also OLIG2 + spheroids generated in a CB1/CB2 dependent manner. (D and E of Fig. 3).

### (2) WIN-derived OLIG2+ progenitor cells differentiated into high purity OPCs expressing PDGFR α, A2B5 and NG2

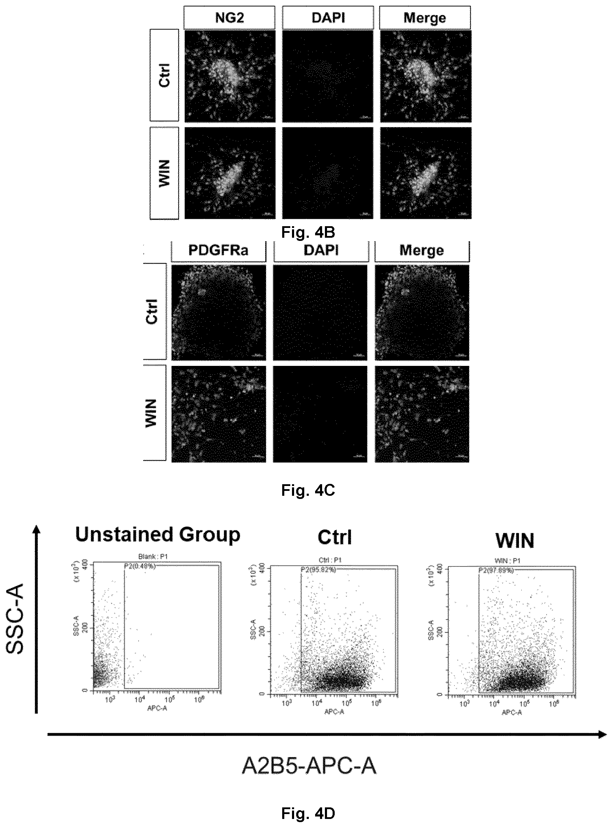

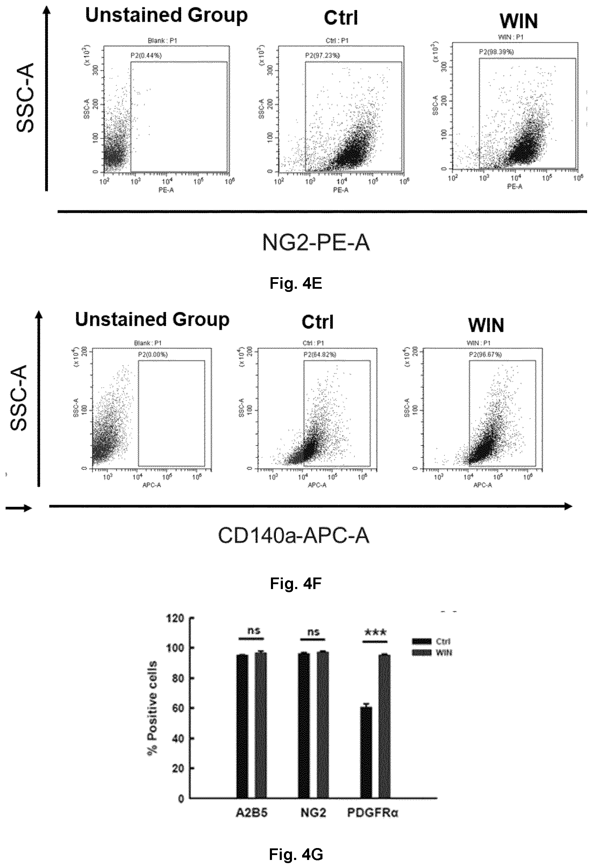

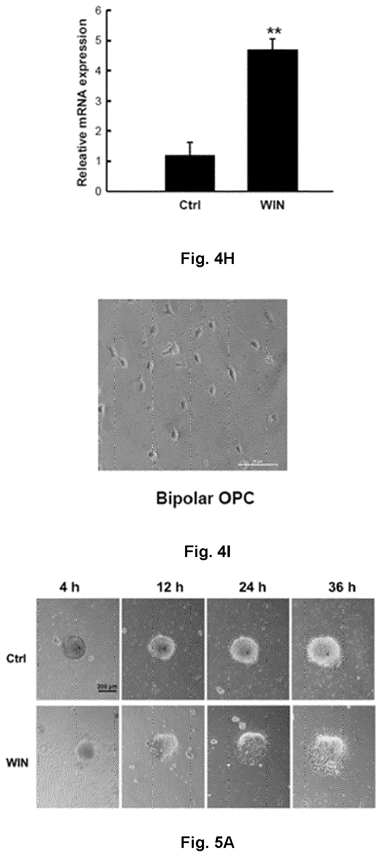

At day 34, upon further differentiation, the inventors performed immunostaining using OPCs cell markers (e.g. PDGFRα, A2B5, and NG2). At the same time, flow cytometry was used to better identify the cell components and the corresponding proportion of positive cells. The results of immunofluorescence and flow cytometry showed that more than 95% of the cells on D34 induced by WIN expressed OPC cell markers NG2 and A2B5. There was no statistically significant difference between the control group and the WIN group (Figs. 4A, 4B, 4D, 4E, and 4G). Interestingly, more than 95.4 ± 2.2% of the cells obtained in the WIN group expressed PDGFR α, which is a widely used molecular marker for OPC, but only 60.7 ± 0.7% in the control group (*n* =3, *P* <0.001, Fig. 4G). Similar results were observed in qRT-PCR. Compared with the control group, the mRNA expression level of PDGFR α in the WIN group was up-regulated (*n* =3, *P* <0.01, Fig. 4H). The typical bipolar OPC image in the WIN group at D34 is shown in Fig. 4I.

### (3) WIN-derived OPCs showed stronger migration ability

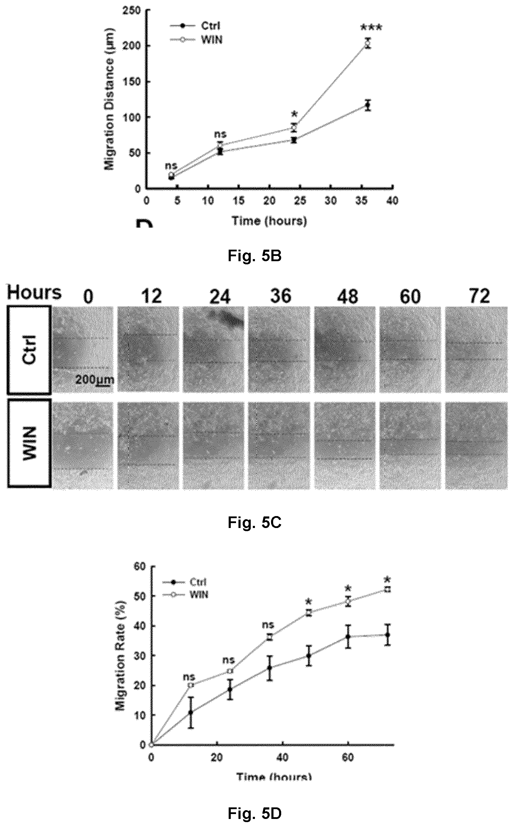

Cell spheres in diameter ranging from 200 to 300 µm were seeded on the GFR Matrigel-coated plate at D30, and the cells migrated out of the spheres cultured with GDM. At 4, 12, 24, and 36 h after seeding, cell spheres and migrated cells were observed and imaged under an inverted microscope (Fig. 5A). The images were analyzed with Image J (i.e. software image J). The results showed that after 36 hours, the average migration distance (203.7 ± 6.3 µm) of cells from WIN-derived cell spheres was significantly longer than that of the control cell spheres (117.2 ± 7.3 µm, *n* =40, *P* <0.001, Fig. 5b). At the same time, the inventor also compared the migration ability of the two groups in the single-cell state. Similar results were observed in the scratch experiment (Fig. 5C). The data showed that at 48h, the migration rate of the WIN group was 44.5 ± 1.0% and that of the control group was 29.9 ± 3.3% (*n* =3, *P* <0.05, Fig. 5D). The above results indicated that WIN-derived OPCs have stronger migration ability.

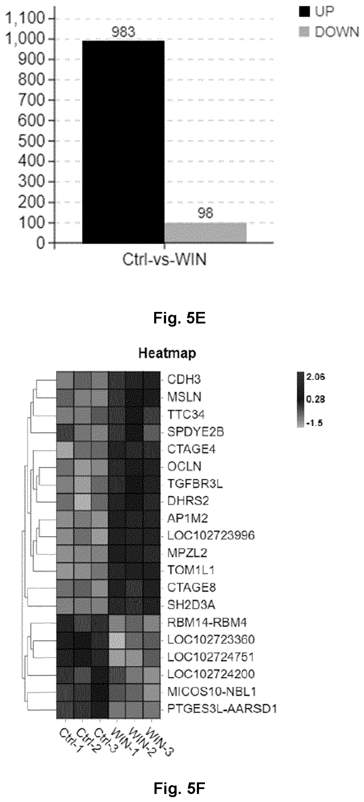

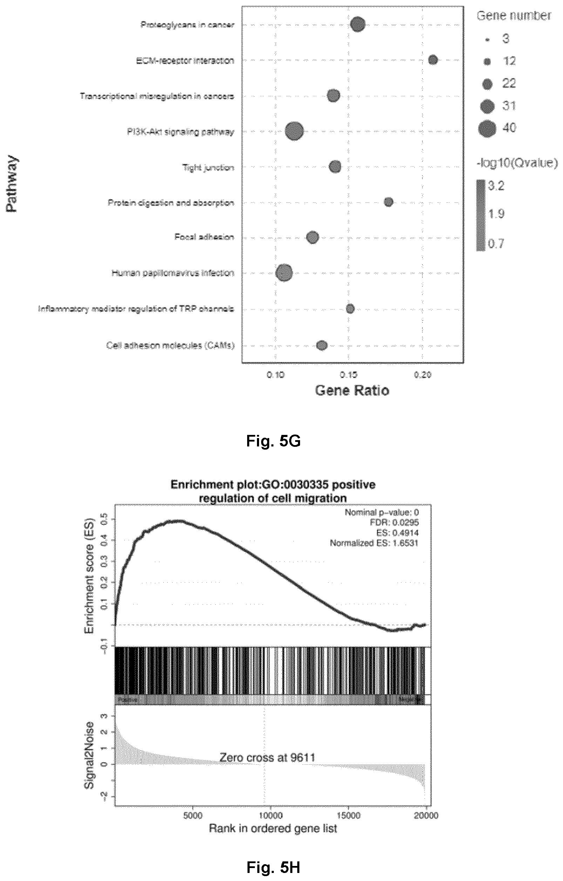

In addition, the inventors performed RNA SEQ on D30 cells. Compared with the control group, 983 genes were up-regulated and 98 genes were down-regulated in the WIN group (Fig. 5E). The heatmap of differential genes is as shown in Fig. 5F. According to the differential genes, the top 10 KEGG pathways, such as PI3K Akt signaling pathway and ECM receptors interact in Fig. 5G. From the GSEA analysis, the biological processes related to cell migration were upregulated in the WIN group compared with the control group (Figure 5H).

### (4) WIN-derived OPCs and mature OLs had high efficiency in vitro.

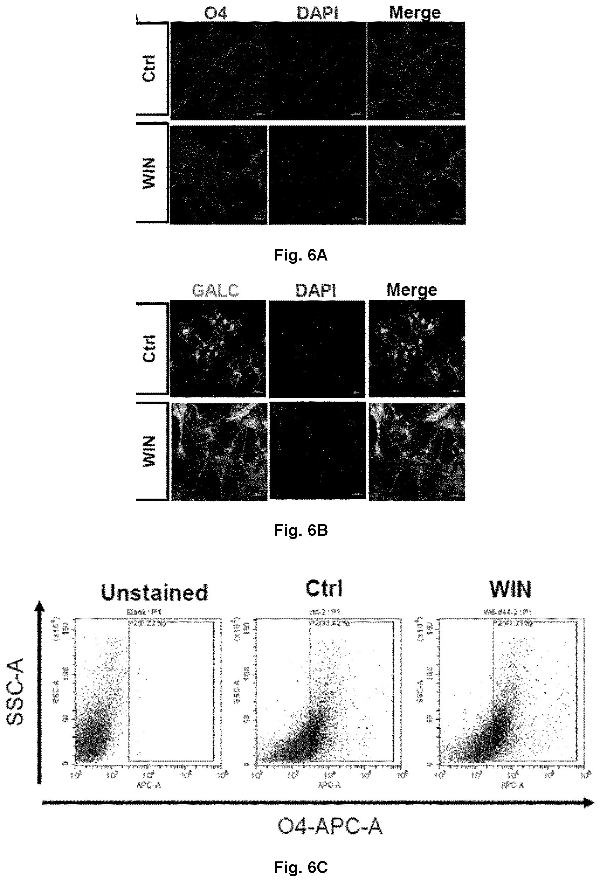

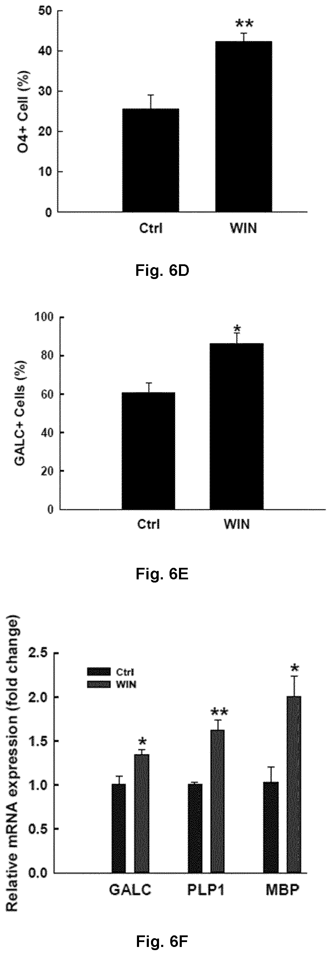

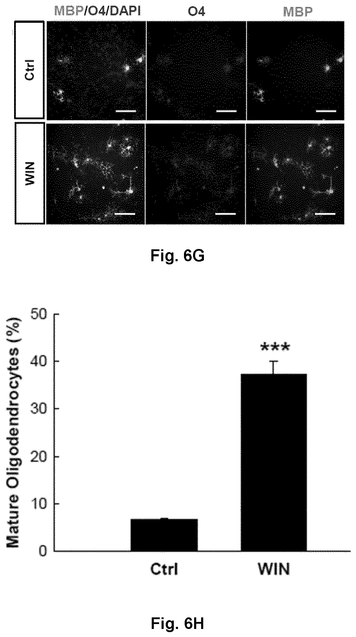

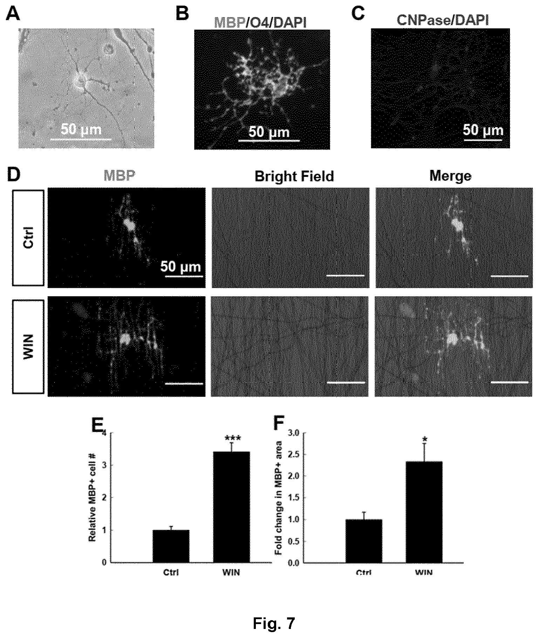

To evaluate the ability of OPC to mature naturally *in vitro,* OPCs were cultured with GDM on GFR (growth factor reduced) Matrigel-coated plates. O4, an advanced OPC marker, was detected at D44 by using immunofluorescence and flow cytometry. The results showed that the percentage of O4+ cells in the WIN group was 42.4 ± 2.1%, which was significantly higher than that in the control group (25.6 ± 3.6%, *n* = 4, *P* <0.01, Figs. 6A, 6B, 6C, and 6D). At D55 of the differentiation timeline, there were more cells expressing immature OL marker galactosylceramidase (GALC) in the WIN group compared with the control group, which were 86.2 ± 5.3% and 60.6 ± 5.3%, respectively (*n* =3, *P* <0.05, Figs. 6B and 6E). The mRNA expression levels of cells at D55 revealed that the mRNA expressions of MBP, GALC and PLP1 were up-regulated in the WIN group (Fig. 6F). At the end of differentiation, more mature OL co-expressing O4 and MBP were detected in the WIN group. The percentages of mature OLs in the WIN group and control group were 37.2 ± 2.9% and 6.6 ± 0.4%, respectively (*n* =3, *P* <0.001, Figs. 6G and 6H). In addition, WIN-derived OPCs could differentiate into branched chain OLs with positive staining for MBP and CNPase (A-C in Fig. 7).

### (5) WIN-derived OLs exerted strong myelination ability in vitro.

To test the myelination ability of WIN-derived OLs *in vitro,* O4 + OPCs classified according to MACS were cultured on a 3D nanofiber scaffolds devoid of neurons, for myelin sheath encapsulation. The 3D nanofiber scaffold contained an electrospun PCL-aligned nanofiber matrix with a diameter of 700 nm, which can simulate the white matter of the brain. After 2 weeks of culture, it was found that the number of MBP+ OLs and the area of nanofibers covered by MBP in the WIN group were significantly increased compared with the control group (D-F in Fig. 7).

### Brief description of the drawings

Fig. 1 shows the timeline of hiPSC differentiation. According to Fig. 1, WIN promoted the generation of OLIG2+ progenitor cells of hiPSC-derived NSC in a CB1/CB2 dependent manner.
Fig. 2 shows an immunostaining diagram of PSC-derived NSCs immunostained with NESTIN, PAX6, and SOX2.
Fig. 3 shows the cell detection diagrams of different treatment groups at D12 and D20 in PSC differentiation culture. Among them, A is a representative immunostaining image of OLIG2 in different treatment groups at D12; ctrl is the control group, Rim is the CB1 inhibitor rimonabant, and Am is the CB2 inhibitor AM630. B is a cell percentage diagram of OLIG2-positive (OLIG2+) in different treatment groups at day 12. C is the relative mRNA expression map of OLIG2 in different treatment groups at D12. D is a representative diagram of cell spheroids at D20 in different treatment groups. E is a diagram of the relative cell sphere number at D20 in different treatment groups. The data mean shown in B, C, and E is the mean ± SEM, *n* =3, P < 0.05 (* *vs*. ctrl; *#vs.* WIN), *P <* 0.001 (### *vs*. WIN).
Fig. 4A shows a representative immunostaining image of WIN-derived OLIG2+ progenitor cells differentiating into OPCs expressing A2B5.
Fig. 4B shows a representative immunostaining image of WIN-derived OLIG2+ progenitor cells differentiating into OPCs expressing NG2.
Fig. 4C shows a representative immunostaining image of WIN-derived OLIG2+ progenitor cells differentiating into OPCs expressing PDGFR α.
Fig. 4D shows a graph of representative flow cytometry data of A2B5+.
Fig. 4E shows a graph of representative flow cytometry data of NG2+.
Fig. 4F shows a graph of representative flow cytometry data of PDGFR α+ (CD140a+).
Fig. 4G shows a graph of cell percentages of A2B5+, NG2+, and PDGFR α+ (CD140a+) in flow cytometry in the control and WIN groups, *n* =3, *P* <0.001 (****vs.* ctrl), ns, no significant difference.
Fig. 4H shows the relative mRNA expression map of PDGFR α in the control group and the WIN group, time is D30, *n* =3, *P* <0.01 (** *vs.* ctrl). The data presented in Fig. 4G and Fig. 4H are mean ± SEM.
Fig. 4I shows an image of typical bipolar OPCs in the WIN group at D34.
Fig. 5A shows representative images of cells migrating out of the spheroids at 4, 12, 24 and 36 h in the control and WIN groups.
Fig. 5B shows the quantification plots of the migration distances of cells from spheroids in the control and WIN populations, *n* = 40, *P* <0.05 (* vs. ctrl), *P* <0.001 (* * * vs. ctrl).
Fig. 5C shows images of representative cell migration of single cells at 0, 12, 24, 36, 48, 60 and 72h after scratching in the control group and WIN group.
Fig. 5D shows plots of the migration rate of the control group and WIN group at 0, 12, 24, 36, 48, 60 and 72 h after scratching, *n* =3, *P* <0.05 (* vs ctrl). The data in Fig. 5B and Fig. 5D are mean ± SEM.
Fig. 5E shows the histogram of differential genes in the WIN group compared with the RNA SEQ group at D30; the up-regulated gene is UP and the down-regulated gene is DOWN.
Fig. 5F shows the heatmap of the top 20 differential genes of the control group and WIN group.
Fig. 5G shows the top 10 pathways enriched in KEGG analysis.
Fig. 5H shows an analytical diagram of the positive regulation of GSEA on cell migration.
Fig. 6A shows images of representative cells at D44 in the control group and WIN group.
Fig. 6B shows images of representative fluorescence stained at D44 in the control group and WIN group.
Fig. 6C shows a diagram of the representative flow cytometry data of O4-APC.
Fig. 6D shows a diagram of the percentage of O4 + cells at D44 in the control group and WIN group in flow cytometry, *n* = 4, *P* <0.01 (** *vs*. ctrl).
Fig. 6E shows a diagram of the representative cells at D55 in the control group and WIN group.
Fig. 6F shows a diagram of the relative mRNA expression of GALC, PLP1 and MBP in the D55 group and WIN group, *n* = 3, *P* <0.05 (* *vs.* ctrl), *P* <0.01 (** *vs.* ctrl).
Fig. 6G shows images of representative fluorescence stained with MBP and O4 at D66 in the control and WIN groups.
Fig. 6H shows a diagram of the percentage of mature OLs at D66 in the control group and WIN group, *n* =3, *P* <0.001 (*** *vs.* ctrl).
Fig. 7 shows diagrams of the *in vitro* myelination detection of WIN-derived OPCs. A is an image of representative cells of WIN-derived mature OLs at D66. B is a representative fluorescence image of WIN-derived mature OLs staining with MBP and O4. C is a representative fluorescence image of WIN-derived mature OL staining with CNPase. D is representative images of MBP immunostaining compared with the control group and WIN group nanofibers. Scale bar: 50 µM. E is a diagram of the relative fold change in the number of MBP+ cells in the control group and WIN group. *n* =3, *P* <0.05 (* *vs.* ctrl), *P* <0.001 (* * * *vs.* ctrl). F is a diagram of the relative fold change of MBP+ area in the control group and WIN group. *n* = 3, *P* < 0.05 (* *vs.* ctrl), *P* < 0.001 (* * * *vs.* ctrl).

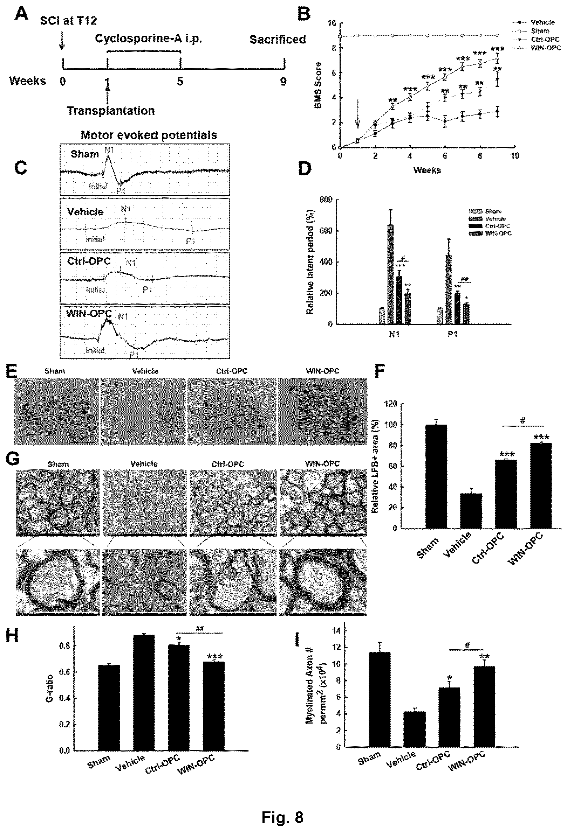

Fig. 8 shows therapeutic effect diagrams of WIN-derived OPCs on SCI mice. The results show that WIN-derived OPCs have better therapeutic effect on SCI mice. Among them, A is the schematic diagram and timeline diagram of cell transplantation in SCI mice. B is the BMS score map of different groups of mice, *n* = 10, *P* <0.01 (***vs.* Vehicle), *P* <0.001 (****vs.* Vehicle). C is the electrophysiological analysis diagram of motor evoked potential (MEP) at the 8th week after transplantation. D is the relative latency map of the N1 peak and P1 peak in different groups. *n* =5, *P* <0.05 (* *vs.* Vehicle; ## vs. ctrl OPC), *P* < 0.01 (* * *vs.* Vehicle; ## *vs.* ctrl OPC), *P* < 0.001 (*** *vs.* Vehicle). E is the representative image of LFB staining at the epicenter of different groups of lesions. Scale bar: 500 µ M. F is the relative LFB+ area map in different groups. *n* = 5, *P* < 0.05 (# *vs.* ctrl OPC), P < 0.001 (* * * *vs.* Vehicle). G is the representative TEM image of the myelin sheath at the epicenter of different groups of lesions. The scale bars in the upper and lower rows are 5 and 1 µM, respectively. H is the quantitative analysis diagram of the myelin G ratio in TEM images of 3 different mice in each group. *P* < 0.05 (* *vs.* vehicle), *P* < 0.01 (## *vs.* ctrl-OPC), *P <* 0.001 (*** *vs.* vehicle). I is a diagram of the number of axons /mm² in the TEM image. *n* = 3, *P* < 0.05 (* *vs.* Vehicle, # *vs.* ctrl-OPC), *P* < 0.01 (** *vs.* Vehicle). Data ± SEM is shown in B, D, E, h, and I.

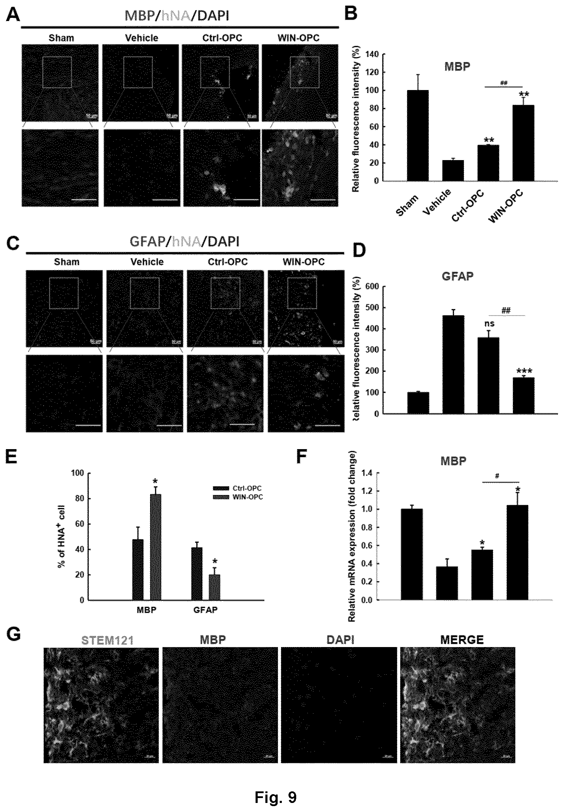

Fig. 9 shows the mature OL detection diagram of WIN-derived OPCs that can differentiate into lesion sites of SCI mice. Among them, A is a series of representative IHC images of MBP (red) and human nuclear hNA (green), and the scale bars on the upper and lower rows are 50 µM. B is the relative fluorescence intensity map of MBP in different groups, *n* =3, *P* <0.01 (** *vs.* Vehicle, ## *vs.* ctrl-OPC). C is a series of representative IHC images of GFAP (red) and human nuclear HNA (green), and the scale bars on the upper and lower rows are 50 µM. D is the relative fluorescence intensity map of GFAP in different groups, *n* =3, *P* <0.01 (## *vs.* ctrl OPC). *P* < 0.01 (*** *vs.* Vehicle). E is the p distribution map of MBP+/GFAP+ cells in HNA+ cells at the lesion site, *n* = 3, *P* <0.05 (**vs.* Vehicle, # *vs.* ctrl-OPC). F is the relative mRNA expression map of MBP in different groups, *n* = 3, *P* <0.05 (* *vs.* Vehicle, #*vs*. ctrl-OPC). G is a series of representative IHC images of mature OLs from WIN-derived OPCs, MBP, and stem121+ (a cytoplasmic protein localized to human cells). The scale bar is 20 µM.

### Detailed description of the invention

The technical solution of the invention is described in detail below in combination with the attached drawings and examples, but the invention is not limited to the scope of the examples.

The experimental methods without indicating specific conditions in the following examples shall be selected according to the conventional methods and conditions or according to the product description. The reagents and raw materials used in the invention are commercially available.

### Definition and interpretation:

WIN55212-2, a CB receptor agonist, was found to be a potent analgesic in rat neuropathic pain models. It activates p42 and p44 MAP kinases via receptor-mediated signaling, which is referred to as WIN for short in the present invention.

RA refers to retinoic acid.

Rimonabant is a CB1 inhibitor, which is referred to as rim for short in the present invention; and AM630 is a CB2 inhibitor, which is referred to as Am for short in the present invention.

Pur in the present invention refers to Purmorphamine, which is the first small molecule agonist developed for smoothened protein. Purmorphamine activates Hedgehog (Hh) signaling pathway, leading to the upregulation and downregulation of its downstream target genes.

The term "PSC" has its common meaning in the art, that is, self-replicating cells that can develop into endoderm, ectoderm and mesoderm cells. Preferably, PSC is hPSC. PSC includes ESC and iPSC, preferably hESC and hiPSC. PSC can be seeded on a surface containing a matrix, such as a gel or a basement membrane matrix.

All culture media and their compositions in the present invention are listed in Table S1:

**Table S1**

| Media | Components |
|---|---|
| mTeSR1 | mTeSR1 basal medium supplemented with 50 ×mTeSR1 supplements and 100 × Penicillin-streptomycin to obtain a final concentration of 1×. |
| Basal medium | DMEM/F12 supplemented with nonessential amino acids (NEAA) 1×, GlutaMAX 2 mM, 2-mercaptoethanol and penicillin-streptomycin, a final concentration of 1×. |
| Neural induction medium (NIM) | Basal medium supplemented with SB431542 (10 µM), LDN193189 (250nM), and RA (0.1 µM) |
| N2 medium | Basal medium supplemented with N2 supplements (Gibco, |
| | USA, 100 × Stock solution), with a final concentration of 1× N2, laminin 4 µg/mL (Roche, Switzerland), bFGF 10 ng/mL (Peprotech, USA), and Noggin 20 ng/mL (Peprotech, USA). RA 0.1 µM (sigma-Aldrich, USA) and 1 µM Purmorphamine (USA) are freshly added at the time of changing medium |
| N2B27 medium | Basal medium containing 1×N2 (Gibco, USA, 100 ×Stock solution) and 1 × B27 (Gibco, USA, 50 ×Stock solution) supplements. |
| PDGF-AA medium | Containing 1 × N2 (Gibco, USA, 100 × Stock solution), 1 × B27 (Gibco, USA, 50 × Stock solution) supplements, biotin 100 ng/mL (sigmaaldrich, USA), PDGF-AA 10 ng/mL (Peprotech, USA), IGF-1 10 ng/mL (Peprotech, USA), T3 60 ng/mL (Sigma-Aldrich, USA), NT3 10 ng/mL (Peprotech, USA), HGF 5 ng/mL (Peprotech, USA), and cAMP 1 µM (Sigma-Aldrich, USA). |
| Glial differentiation medium (GDM) | Containing 1 × N2, 1 × B27 supplement, biotin 100 ng/mL (Sigma-Aldrich, USA), T3 60 ng/mL (Sigma-Aldrich, USA), ascorbic acid 20 µg/mL (Sigma-Aldrich, USA), NT3 10 ng/mL (Peprotech, USA), HEPES 10 mM (Sigma-Aldrich, USA), cAMP 1 µM (Sigma-Aldrich, USA), Y-27632 10 µM (Cayman, USA) and Heregulin 10 ng/mL (Peprotech, USA). |

The primary and secondary antibodies used for immunofluorescence staining in the present invention (Tables S2 and S3):

**Table S2**

| Primary antibody | Company | Catalog Nos |
|---|---|---|
| Rabbit anti-Olig2 | abcam | ab254043 |
| Rabbit anti-PDGFRa | CST | 3174 |
| Rabbit anti-NG2 | abcam | ab129051 |
| Rabbit anti-MBP | CST | 78896 |
| Mouse anti-A2B5 | Invitrogen | 433110 |
| Rabbit anti-SOX2 | Cell Signaling | 3579S |
| Mouse anti-NESTIN | Cell Signaling | 33475 |
| Rabbit anti-PAX6 | Cell Signaling | 60433 |
| Mouse anti-GFAP | Bioworld | MB9017 |
| Rabbit anti-NeuN | abcam | ab177487 |
| Mouse anti-NeuN | NOVUS | NBP1-92693SS |
| Mouse anti-NKX2.1 | Millipore | MAB5460 |

**Table S3**

| | | |
|---|---|---|
| Donkey anti-mouse 594 | abcam | ab150108 |
| Donkey anti-rabbit 594 | Invitrogen | 1987293 |
| Goat anti-mouse 488 | abcam | ab150117 |
| Goat anti-rabbit 488 | abcam | ab150077 |
| Sheep anti-mouse 594 | abcam | ab6806 |
| Goat anti-chicken 488 | abcam | ab150173 |
| Donkey anti-rabbit 647 | abcam | ab150075 |

The primer sequences of the target genes of real-time fluorescent quantitative PCR in the present invention:

**Table S4**

| target genes | Upstream primers | Downstream primers |
|---|---|---|
| GLI1 | AGCGTGAGCCTGAATCTGTG (SEQ ID NO:1) | CAGCATGTACTGGGCTTTGAA (SEQ ID NO:2) |
| OLIG2 | CCAGAGCCCGATGACCTTTT (SEQ ID NO:3) | TCCGGCTCTGTCATTTGCTT (SEQ ID NO:4) |
| MBP | CCGGCAAGAACTGCTCACTA (SEQ ID NO:5) | CGTCTAGCCATGGGTGATCC (SEQ ID NO:6) |
| NESTIN | GAAGGGCAATCACAACAGGTG (SEQ ID NO:7) | GGGGCCACATCATCTTCCA (SEQ ID NO:8) |
| PATCH1 | GAAGAAGGTGCTAATGTCCTGAC (SEQ ID NO:9) | GTCCCAGACTGTAATTTCGCC (SEQ ID NO:10) |
| PLP1 | ACCTATGCCCTGACCGTTG (SEQ ID NO:11) | TGCTGGGGAAGGCAATAGACT (SEQ ID NO:12) |
| PDGFR | TTGAAGGCAGGCACATTTACA (SEQ ID NO:13) | GCGACAAGGTATAATGGCAGAAT (SEQ ID NO:14) |
| SMO | TCGAATCGCTACCCTGCTG (SEQ ID NO:15) | CAAGCCTCATGGTGCCATCT (SEQ ID NO:16) |
| SOX10 | CCTCACAGATCGCCTACACC (SEQ ID NO:17) | CATATAGGAGAAGGCCGAGTAGA (SEQ ID NO:18) |

### Example 1

### 1.1 Cell culture and cell differentiation

The human induced pluripotent stem cell (hiPSC) line (UE017C1) from Guangzhou Institute of Biomedicine and Health, Chinese Academy of Sciences, was cultured on Matrigel-coated six-well plates using mTeSR^{™} (Stem Cell Technologies, Canada) in a 37 °C incubator with 5% CO₂.

In order to generate PAX6+/NESTIN+/SOX2+ NSC, the dual SMAD neural induction method was used. hiPSCs with a density of -80% with neural induction medium (NIM) were treated from day 0 (D0) to day 7, and the medium was changed every day. Starting from D8, the medium was changed daily to fresh N2 medium containing RA (100nM), purine amine (PUR, 1 µM) and WIN (1 µM). Starting from D12, cells were manually isolated and suspended in the ultra-low attachment plate in N2B27 medium containing RA (100nM), PUR (1 µM) and WIN (1 µM), and the medium was changed every other day. Starting from D20, the spheroids were further suspended in PDGF-AA medium for culture. At D30, expanded spheroids were seeded on Matrigel-coated plates or Petri dishes with low growth factors in the glial differentiation medium (GDM). All medium and their compositions are listed in Table S1. Fig. 1 shows the timeline of the culture process of the present invention.

It should be understood that the PAX6+/NESTIN+/SOX2+ NSC described in the present invention is not limited to those obtained by induced differentiation with hiPSCs, but can also be directly obtained from commercially available products, and further cultured according to the method described in the present invention to obtain oligodendrocyte precursor cells (OPCs) and oligodendrocytes (OLs) for the treatment of spinal cord injury (SCI).

The above-mentioned human induced pluripotent stem cells (hiPSCs) can also be replaced by human embryonic stem cells (hESCs), which are established cell lines that can proliferate infinitely and do not involve the application of human embryos for industrial or commercial purposes, such as H9 human embryonic stem cell lines.

### 1.2 Immunofluorescence staining

The identification of cell markers in the present invention uses an immunofluorescence staining method, wherein cells (NSCs, OPCs and OLs) at different culture stages were cultured on confocal Petri dishes coated with Matrigel or Matrigel with reduced growth factors, washed three times with pre-cooled PBS, and fixed with 4% paraformaldehyde (PFA) at room temperature for 15 minutes. The fixed samples were then washed three times, permeabilized and blocked with blocking solution containing 0.5% triton X-100 and 2% bovine serum albumin (BSA) for 30 min at room temperature. The diluted primary antibodies listed in Table S2 were applied to cover the cells overnight at 4 °C. The next day, the corresponding secondary antibodies (listed in Table S3) were added for staining in the dark for 60 min at room temperature after washing. DAPI containing anti-fluorescence quencher was then added to the culture dish for nuclear staining. Images were taken using a confocal microscope (Zeiss, LSM880) or an inverted microscope (Nikon, Eclipse Ti2-U).

### 1.3 Fluorescence quantitative real-time PCR (qRT-PCR)

The present invention uses qRT-PCR to calculate the relative mRNA expression fold changes. The specific method is as follows: according to the manufacturer's instructions, the RNA rapid purification kit (ES science, Shanghai, China) was used to collect and purify the total RNA of cells and spinal cord tissue. After quantifying the RNA concentration with NANODROP ONE (Thermo Fisher Scientific, USA), EasyScript^{®} Integrated first-line cDNA synthesis Supermix and T100TM thermal cycler (BIO-RAD) was used for qPCR (one-step gDNA removal), and then PerfectStart Green qPCR SiperMix (TRNAS, China) and LightCycler^{®} 96 (Roche) were used for quantification by qRT-PCR. All processes were performed according to the manufacturer's instructions. 2 ^{- ΔΔCt} method was used to calculate the relative mRNA expression fold changes. The primer sequences of all target genes are listed in Table S4.

### 1.4 Cell migration ability test

In order to test the migration ability, the inventor carried out experiments on two dimensions of spheroid and single-cell state. First, the inventors have observed the ability of cells in the control and WIN groups to migrate out of the spheroids. Selected spheroids of the same size at day 30 from the control and WIN groups were seeded on 6-well plates coated with Matrigel with reduced growth factor in GDM medium. After 4, 12, 24, and 36 h, cell spheres and migrated cells were observed and imaged under an inverted microscope.

In the scratch assay, 34-day-old cells were digested with pre-warmed accutase and filtered through 40 µM cell filter to produce a single-cell suspension. After centrifugation, these single cells were seeded on a 24-well plate coated with Matrigel with reduced growth factor at the density of 2 ×10⁶/ mL and cultured with GDM. When the cell confluence reached 70-80%, a scratch was formed on the cell using 10 µL the pipette tip. After washing with DPB, fresh GDM was added to the wells. Cell images were collected at 0, 12, 24, 36, 48, 60, and 72 h using an inverted microscope. All images were measured and analyzed by Image J (i.e., software image J).

### 1.5 Flow cytometry

Taking flow cytometry based on anti-O4-APC antibody as an example, 2 µL antibody was added to a 98 µL of single cell suspension containing 1 × 10⁶ cells and cell staining buffer, mixed well and incubated for 10 min in the dark in a refrigerator. After incubation, cells were washed and dried. The cell pellet was then resuspended in an appropriate amount of buffer and filtered through 40 µM cell filter prior to analysis. Flow cytometry was performed on a CytoFLEX cytometer (Beckman, USA). The primary antibodies for flow cytometry are listed in Table S2.

### 1.6 MACS cell isolation

O4 + OPC isolation was performed according to the manufacturer's instructions using MACS based on anti-O4 microbeads (MiltenyiBiotec, Germany). A single cell suspension was prepared by digesting cells with pre warmed Accutase at 37 °C for 30 min at day 44. After digestion, the cell mixture was diluted with pre-cooled DMEM/F12 and centrifuged at 300g for 5 min at room temperature. The cells were then resuspended in DMEM/F12 and filtered through 40 µM cell filter, and cells were collected by centrifugation at 300g for 5 min at room temperature. After mixing evenly, the cell mixture was incubated in a refrigerator for 15 min. Then 1 mL of cold MACS buffer was added and centrifuged at 300g for 10 min. The cell pellet was resuspended in 500 µL cold MACS buffer and mixed well. Afterwards, the cell mixture was added to the MS column and pre-rinsed using 500 µL cold MACS buffer in the magnetic field of a suitable MACS separator, and the column was washed three times with 500 µL buffer. Unlabeled cells were collected from the effluent. When the column reservoir was empty, the column was placed on a 15 mL collection tube and rinse with 1 mL buffer. Magnetically labeled cells were collected from the effluent by firmly pushing the plunger into the column. The eluted O4+ rich cells were then centrifuged at 300g for 5 min and resuspended in 1mL GDM.

### 1.7 Nanofiber myelination assay

Nanofiber chamber slides aligned with electrospun polycaprolactone (PCL) with a diameter of 700nm were purchased from Sigma-Aldrich and coated with Matrigel with growth factor at 37 °C overnight. O4 + OPCS sorted by MACS were seeded in each chamber at 5 × 10⁴ cells, and 1mL of GDM was added, and the medium was changed every 2 days. After 3 weeks of culture, cells were fixed, blocked, and stained as described in the immunofluorescence section. Images were taken by a confocal microscope (Zeiss, LSM880).

### 1.8 RNA sequencing

Total RNA was extracted using RNA rapid purification kit (ES science, Shanghai, China) according to the manufacturer's instructions. RNA library sequencing was performed on an Illumina HiseqTM 2500/4000 by Gene Denovo Biotechnology Co., Ltd. (Guangzhou, China). Bioinformatics analysis was performed using Omicsmart, which is a real-time interactive online platform for data analysis ( http://www.omicsmart.com ).

Establishment of a spinal cord injury model and validation of the therapeutic effect of cell transplantation:

### (1) The spinal cord injury model

Female C57BU6 mice were anesthetized by injecting intraperitoneally 1.25% tribromoethanol at a dose of 300 µL per 20g, and the hair was shaved around T12, which is the highest point on the back of mice. The skin around T12 was cut about 1.0 cm to expose the paraspinal muscles on both sides of the spine and the dura mater of the spinal cord. The mice were fixed in a special spinal cord groove and received a hit by dropping a 10 g weight rod from a height of 6.25 mm using a spinal cord impactor (New York University impactor model II). After hemostasis, the deep fascia, subcutaneous tissue, and skin of the mice were sequentially sutured with a surgical needle. After disinfection of the wound with Iodophor, each mouse was injected intraperitoneally with normal saline containing 150 µL of gentamicin. The consistency of the degree of injury between mice was ensured by monitoring the settings of the impactor (such as contusion impact velocity and compression rate). The mice were then placed on a small animal heating pad during recovery from anesthesia. After the operation, the animals were manually urinated three times per day until they urinated independently. Gentamicin injection was given daily in the first week after SCI to prevent infection.

### (2) Cell transplantation

More than 95% of the cells in the control group and WIN group of D34 were NG2 and A2B5 positive. SCI mice received OPC or vehicle (saline) transplantation at day 8 after SCI. Under anesthesia, the spinal cord was re-exposed and a small hole was opened in the dura above the injury center. 1 × 10⁵ cells suspended in 5 µL salt water were injected into the lesion site of SCI mice within 30 seconds. After injection, the microsyringe was left at the injection site for 60 seconds and slowly withdrawn to avoid the outflow of the cell suspension. For immunosuppression, mice were given cyclosporine-A (10mg/kg) intraperitoneally every day from one day before cell transplantation and continued until the fourth week after transplantation.

### (3) Basso mouse scale

Motor function recovery of the hind limbs was assessed by the Basso mouse scale (BMS). In brief, two independent raters were trained with BMS recordings and were blinded to the grouping of mice throughout the experiment. Postoperative examinations were performed at days 1 and 7 after SCI, and then weekly until the eighth week after transplantation. Each mouse was scored according to scale criteria by walking freely in an open field for 4 minutes and using a 0 to 9 point scoring system.

### (4) Electrophysiology

Eight weeks after cell transplantation, motor evoked potentials (MEP) were recorded by using Powerlab/15 T (ADInstruments, Inc., Australia). Briefly, after complete anesthesia with 1% sodium pentobarbital, the silver electrodes were placed close to the right cortical sensorimotor area and the right sensorimotor cortex of mice. At the same time, bipolar recording electrodes were placed on the surface of the contralateral sciatic nerve. The frequency of the pulse generator was set at 4 Hz, the intensity was set at 4.5 mA, and the duration was 0.2 ms, to generate stimuli. The analog signal of MEP was amplified 100,000 times with an average of 256 scans. The interval periods between N1 wave and P1 wave at the electromechanical station were recorded and analyzed.

### (5) Immunohistochemistry

After deep anesthesia with 1% sodium pentobarbital, the mice were electrophoretically infused with PBS prepared with 0.1M phosphate-buffered saline and 4% paraformaldehyde 8 weeks after transplantation. The dissected spinal cord was fixed in 4% PFA for 24 h, then gradient dehydrated in 15% and 30% sucrose solution at 4 °C and embedded in OCT (Sakura fine Technology Co., Ltd., Tokyo, Japan). Embedded spinal cord tissue was cryosectioned using a cryostat (CRYOSTAR NX50, Thermo), with a thickness of 8-10 µm.

For IHC, frozen sections were thawed in advance, washed three times with PBS, blocked with blocking solution containing 3% BSA and 0.3% triton X-100 in PBS for 60 min at 37 °C, and incubated with primary antibodies overnight at 4 °C. The next day, the sections were rinsed three times with cold PBS and incubated with secondary antibodies for 1 h at room temperature in the dark. The slides were then covered with mounting solution containing DAPI and sealed with nail polish. Images were collected using a confocal microscope (Zeiss, LSM880).

### (6) Luxol fast blue (LFB) staining

Fresh spinal cord tissue was fixed with 4% paraformaldehyde for more than 24 hours, then dehydrated with gradient alcohol (90%, 95%, absolute ethanol), alcohol benzene and xylene, and then waxed. Waxed tissues were embedded in the embedding machine and trimmed. The trimmed paraffin blocks were cut to a thickness of 4 µm fragments using a microtome (Leica). After dewaxing and washing, the sections were incubated with pre-warmed myelin staining solution A for 1 h. Then the sections were immersed in myelin staining solution B for 2 seconds and directly immersed in myelin staining solution C for 15 seconds, and the staining process was terminated by washing with water. After counterstaining with eosin, sections were dehydrated with absolute ethanol and xylene, and mounted with neutral glue. Images were collected using an inverted microscope (Nikon, Eclipse Ti2-U).

### (7) Transmission electron microscope

The spinal cord tissue removed from the injury site was trimmed to 1 mm × 1 mm × 1 mm, placed in the electron microscope fixing solution, kept at 4 °C for 2-4 hours, washed three times with 0.1M PBS (pH 7.4) again, and fixed in 0.1M PBS containing 1% osmonic acid for 2h at room temperature. After fixation and washing, the tissue was dehydrated with gradient alcohol (50%-70%-80%-90%-95%-100%-100%) and acetone. Then the tissue was permeated with a combination of acetone and 812 embedding agent at a ratio of 1:1 and 2:1 for 2-4h and overnight, respectively. Afterward, the tissue was permeated with 812 embedding agents for 5-8 h. The tissue was then inserted into an embedding plate containing 812 embedding agents, and left in a 37 °C oven overnight and polymerized in a 60 °C oven for 48 h. Sections were stained with 2% uranyl acetate saturated alcohol solution for 15 min, lead citrate for 15 min, and dried overnight at room temperature. TEM images were observed and collected using a transmission electron microscope (HT7700, HITACHI).

### (8) Statistical analysis

Statistical significance analysis was performed using unpaired t-test for two sample comparisons, or one-way ANOVA with post hoc Bonferroni test for three sample comparisons. Significant differences were shown as P <0.05 (*, #), *P* <0.01 (**, ##), and *P* <0.001 (***, ###). All experiments were performed independently at least 3 times. All data are shown as mean ± SEM.

### Example 2: WIN-derived OPC transplantation improves motor recovery in SCI mice

In order to investigate the potential of WIN-derived OPC as SCI treatment, the inventors transplanted OPCs into the lesions of SCI mice and assessed motor function using the BMS scale, as shown in Figs. 8A and 8B. After SCI, mice were paralyzed and lost hindlimb mobility (BMS score = 0). On the seventh day after SCI, OPCs in the WIN group or control group were radiated to the lesion site of SCI. The vehicle group received the same volume of saline at the lesion site. After cell transplantation, the mice gradually recovered function in the hind limbs. From the second week after transplantation, the motor function of the hind limb in the WIN group was significantly improved compared with that in the vehicle group. In the control group, the motor function of the hind limbs of mice was improved significantly after the fourth week of cell transplantation, which was significantly later than that in the WIN group. In addition, compared with the control OPCs transplanted mice, the BMS score of mice transplanted with WIN-derived OPCs increased significantly from 2 weeks after transplantation to the last day of the behavioral test (n = 10, *P* <0.01). After the 8th week of cell transplantation, the recovery rate of WIN group mice was significantly better than that of the control group, and the corresponding BMS scores were 7.2 ± 0.4 and 5.5 ± 0.6, respectively (*n* = 10, *P* <0.05, Fig. 8B).

To further evaluate the recovery of motor function, the inventors performed the electrophysiological analysis of MEP in SCI mice 8 weeks after transplantation (Fig. 8C). The relative late t-cycles of N1 peak and P1 peak in both the control OPC and WIN-OPC transplantation groups were shorter than those in the vehicle group. Compared with the control OPC transplantation group, the relative late t-cycles of the N1 peak and P1 peak in the WIN-OPC transplantation group were significantly shortened (Fig. 8D). *n* = 5, *P <* 0.05 (* *vs.* vehicle; # *vs.* ctrl OPC), *P* < 0.01 (** *vs.* vehicle; ## *vs.* ctrl-OPC), *P <* 0.001 (*** vs. vehicle). The above results indicate that WIN-derived OPCs have a better therapeutic effect on SCI by enhancing motor recovery.

### Example 3: Transplantation of WIN-derived OPCs into SCI mice can improve their demyelination

To demonstrate the myelin sheath at the epicenter of the lesion in each group of mice, the inventor performed LFB staining on coronal sections of the spinal cord. The results showed that compared with the sham group, the area s of LFB+ in the vehicle group was significantly reduced, indicating that demyelination occurred after SCI (Figs. 8E and 8F). Although the implantation of OPCs into the lesion site could increase LFB + area, improve demyelination and maintain tissue integrity, the LFB+ area of the WIN group was larger than that of the control group, indicating that WIN-derived OPCS had better remyelination effects compared with control OPCs (Figs. 8E and 8F), *n* = 5, *P* < 0.05 (# *vs.* ctrl-OPC), *P <* 0.001 (*** *vs.* vehicle).

In order to observe myelinated axons at the lesion site more microscopically, the inventor observed them by transmission electron microscopy (TEM). First, the number of myelinated axons was counted. As shown in Figs 8G and 8H, axons in the vehicle group experienced a great loss compared with the sham group. Compared with vehicle mice, mice receiving OPC transplantation had an increased number of myelinated axons around the lesion site. Moreover, the number of myelinated axons per mm² in WIN-OPC was significantly higher than that in control OPC, which were 7.15 ± 0.74 and 9.70 ± 0.78, respectively. *n* = 3, *P* < 0.05 (* *vs.* vehicle, # *vs.* ctrl-OPC), *P* < 0.01 (** *vs.* vehicle).

In addition, the thickness of the myelin sheath can be estimated by measuring the g ratio, which is the ratio of inner axon diameter to outer diameter. As shown in Figs. 8G and 8H, the greater the g ratio is, the thinner the myelin sheath is. The g ratio of the vehicle group was 0.88 ± 0.01, which was larger than that of other groups, indicating that the remaining axons in SCI mice were undergoing demyelination. Compared with vehicle mice, mice receiving OPC transplantation had more myelinated axons and thicker dense myelin sheaths. Transplantation of exogenous OPCs can alleviate the demyelinating process of SCI. However, a more dense myelin sheath was observed in the WIN-OPC group. The g ratio of the WIN-OPC group was 0.67 ± 0.02, which was lower than that of the control OPC group (0.81 ± 0.02, *n* =3, *P* <0.01), indicating that the therapeutic effect of WIN OPCs was better than that of control OPCs. *P* < 0.05 (* *vs.* vehicle), *P* < 0.01 (##*vs*. ctrl OPC), *P* < 0.001 (*** *vs.* vehicle).

### Example 4: WIN-derived OPCs can distinguish functional OLs involved in remyelination.

To determine the fate of transplanted cells, IHC with anti-human nuclear (HNA) antibody was used to track them. IHC of sagittal frozen sections of the spinal cord revealed that most HNA-positive cells in the WIN-OPC group could be stained with anti MBP antibody (Figs. 9A and 9E). The percentage of HNA/MBP double positive cells in WIN-OPC group was 83.2 ± 5.9%, which was significantly higher than 47.7 ± 9.9% in control OPC group (*n* = 5, *P* <0.01, Fig. 9E). The percentage of HNA/GFAP double-positive cells in the control group and WIN group were 41.3 ± 4.4% and 21.2 ± 6.5%, respectively (*n* = 5, *P* <0.01, Figs. 9C and 9E). After transplanting WIN-derived OPCs into the spinal cord of SCI mice, most cells differentiated into OLs. Under the same capturing conditions, the relative average fluorescence intensity of MBP in the WIN group was significantly stronger than that in the control group, while the relative average fluorescence intensities of GFAP in the two groups were opposite (*n* =3, *P* <0.01, 9b and 9D). The relative mRNA expression of MBP in the WIN-OPC group was higher than that in the control OPC group (*n* = 3, *P* <0.05, 9F). In addition, STEM121 (green) (C-type cytosolic protein specific for human cells) and MBP (red) were used to visualize human myelin OLs in SCI mouse spinal cord tissue. The fluorescence image in Fig 9G shows that WIN-derived OPCs can differentiate into branched OLs *in vivo* and integrate into the spinal cord for remyelination.

The above examples are the preferred embodiments of the present invention, but the embodiments of the present invention are not limited by the above examples. Any other changes, modifications, substitutions, combinations, and simplifications that do not deviate from the spirit and principles of the present invention should be equivalent replacement methods and are included in the scope of protection of the present invention.

## Claims

1. A method for preparing OLIG2+ neural stem cells, which is **characterized by** comprising the following steps:
(1) generating NSCs of PAX6+/NESTIN+/SOX2+: PSCs were cultured with neural induction medium from day 0 to day 7, and the medium was changed every day; and
(2) from day 8, changing the medium to fresh N2 medium containing retinoic acid, Purmorphamine and CB receptor agonist every day for 4-5 days.

2. A method for producing oligodendrocyte precursor cells, which is **characterized by** comprising the following steps:
(1) generating NSCs of PAX6+/NESTIN+/SOX2+: PSCs were cultured with neural induction medium from day 0 to day 7, and the medium was changed every day;
(2) from day 8, changing the medium to fresh N2 medium containing retinoic acid, Purmorphamine and CB receptor agonist every day;
(3) from day 12, manually isolating the cells and suspending them in N2B27 medium containing retinoic acid, Purmorphamine and CB receptor agonist, and changing the medium every other day;
(4) from day 20, further suspending spheroids in PDGF-AA medium;
(5) from day 30, seeding and culturing expanded spheroids on Matrigel-coated plates or Petri dishes with low growth factors in glial differentiation medium for 3-15 days.

3. A method for producing oligodendrocytes, which is **characterized by** comprising the following steps:
(1) generating NSCs of PAX6+/NESTIN+/SOX2+: PSCs were cultured with neural induction medium from day 0 to day 7, and the medium was changed every day;
(2) from day 8, changing the medium to fresh N2 medium containing retinoic acid, Purmorphamine and CB receptor agonist every day;
(3) from day 12, manually isolating the cells and suspending them in N2B27 medium containing retinoic acid, Purmorphamine and CB receptor agonist, and changing the medium every other day;
(4) from day 20, further suspending spheroids in PDGF-AA medium;
(5) from day 30, seeding and culturing expanded spheroids on Matrigel-coated plates or Petri dishes with low growth factors in glial differentiation medium for 16-36 days.

4. The method according to any one of claims 1-3, **characterized in that** the step (1) is replaced with the NSC finished product of PAX6+/NESTIN+/SOX2+.

5. The method according to any one of claims 1-3, **characterized in that** the PSCs are cultured on Matrigel-coated six-well plates using mTeSR^{™} medium in a 37°C incubator with 5% CO₂.

6. The method according to any one of claims 1-3, **characterized in that** the concentration of the retinoic acid is 10-1000nM, the concentration of the Purmorphamine is 0.1-10 µM, and the concentration of the CB receptor agonist is 0.1-10 µM.

7. The method according to any one of claims 1-3, **characterized in that** the concentration of the retinoic acid is 100 nM, the concentration of the Purmorphamine is 1 µM, and the concentration of the CB receptor agonist is 1 µM.

8. The method according to any one of claims 1-3, **characterized in that** the CB receptor agonist is selected from one or more of CP55940, AM-1241, JVH-015, JVH-151, JVH-120, A-796260, L-768242, resunab, Arachidonylcyclopropylamide, 2-Arachidonoyl glycerol-d8, Bay 59-3074, BML-190, GW842166X, Noladin ether and Gp 1a.

9. The method according to any one of claims 1-3, **characterized in that** the CB receptor agonist is WIN55212-2.

10. An oligodendrocyte precursor cell, **characterized in that** it is prepared according to the method of claim 2.

11. An oligodendrocyte, **characterized in that** it is prepared according to the method of claim 3.

12. Use of the oligodendrocyte precursor cell of claim 10 or the oligodendrocyte of claim 11 in the preparation of medicament for the treatment of demyelinating and myelin sheath injury diseases;
the demyelinating diseases include spinal cord injury, stroke, multiple sclerosis, neuromyelitis optica, Guillain-Barre syndrome, diffuse disseminated cerebrospinal meningitis, acute disseminated encephalomyelitis, concentric sclerosis, diffuse sclerosis, leukodystrophy, leukoencephalopathy caused by ischemia-hypoxia disease, central pontine myelinolysis, acute inflammatory demyelinating polyneuropathy, chronic inflammatory demyelinating polyneuropathy, subacute combined degeneration caused by nutritional deficiency disease, subacute sclerosing panencephalitis or progressive multifocal leukoencephalopathy caused by viral infection, diabetic neuropathy, neuropathy of systemic lupus erythematosus, white matter ablation disease, adrenoleukodystrophy, and pelizois metzbach disease;
the myelin sheath injury diseases include Down syndrome, Alzheimer's disease, and Parkinson's disease.

13. A medium for promoting neural stem cell differentiation, the components of which comprise: fresh N2 medium containing retinoic acid, Purmorphamine and CB receptor agonist.

14. The medium according to claim 13, **characterized in that** the concentration of the retinoic acid is 10-1000nM, the concentration of the Purmorphamine is 0.1-10 µM, and the concentration of the CB receptor agonist is 0.1-10 µM.

15. The medium according to claim 13, **characterized in that** the concentration of the retinoic acid is 100 nM, the concentration of the Purmorphamine is 1 µM, and the concentration of the CB receptor agonist is 1 µM.

16. A medium for promoting neural stem cell differentiation, the components of which comprise fresh N2B27 medium containing retinoic acid, Purmorphamine and CB receptor agonist.

17. The medium according to claim 16, **characterized in that** the concentration of the retinoic acid is 10-1000nM, the concentration of the Purmorphamine is 0.1-10 µM, and the concentration of the CB receptor agonist is 0.1-10 µM.

18. The medium according to claim 16, **characterized in that** the concentration of the retinoic acid is 100 nM, the concentration of the Purmorphamine is 1 µM, and the concentration of the CB receptor agonist is 1 µM.

19. Use of the medium according to any one of claims 13-18 for promoting neural stem cell differentiation.
